(19) Europäisches Patentamt
European Patent Office
Office européen des brevets

(11) **EP 4 726 334 A1**

(12) **EUROPEAN PATENT APPLICATION**

(43) Date of publication:
**15.04.2026 Bulletin 2026/16**

(21) Application number: **25203895.5**

(22) Date of filing: **23.09.2025**

(51) International Patent Classification (IPC):
**G01D 5/353** $^{(2006.01)}$

(52) Cooperative Patent Classification (CPC):
**G01D 5/35364**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **08.10.2024 JP 2024176767**

(71) Applicant: **Yokogawa Electric Corporation Tokyo 180-8750 (JP)**

(72) Inventors:
- **Watanabe, Fumie**
  **Musashino-shi, Tokyo, 180-8750 (JP)**
- **Suzuki, Yuta**
  **Musashino-shi, Tokyo, 180-8750 (JP)**
- **Tezuka, Shin-ichirou**
  **Musashino-shi, Tokyo, 180-8750 (JP)**
- **Honma, Masayoshi**
  **Musashino-shi, Tokyo, 180-8750 (JP)**

(74) Representative: **Stöckeler, Ferdinand et al Schoppe, Zimmermann, Stöckeler Zinkler, Schenk & Partner mbB Patentanwälte Radlkoferstrasse 2 81373 München (DE)**

(54) **OPTICAL FIBER CHARACTERISTIC MEASUREMENT SYSTEM AND HUMIDITY MEASUREMENT METHOD**

(57) An optical fiber characteristic measurement system (1, 2, 3) according to the present disclosure includes a first optical fiber (21), a second optical fiber (22) having refractive index characteristics different from those of the first optical fiber (21), and an optical fiber characteristic measurement apparatus (10) configured to measure a first Brillouin frequency shift, which is a Brillouin frequency shift of Brillouin scattered light from the first optical fiber (21), and a second Brillouin frequency shift, which is a Brillouin frequency shift of Brillouin scattered light from the second optical fiber (22). The optical fiber characteristic measurement apparatus (10) is configured to measure humidity at a predetermined position based on the first Brillouin frequency shift and the second Brillouin frequency shift.

*FIG. 1*

**Description**

CROSS-REFERENCE TO RELATED APPLICATION

The present application claims priority to Japanese Patent Application No. 2024-176767 filed on October 8, 2024, the entire contents of which are incorporated herein by reference.

TECHNICAL FIELD

The present disclosure relates to an optical fiber characteristic measurement system and a humidity measurement method.

BACKGROUND

An example of a known optical fiber characteristic measurement apparatus is an optical fiber characteristic measurement apparatus that measures the characteristics of an optical fiber by Brillouin Optical Correlation Domain Reflectometry (BOCDR).

The BOCDR type optical fiber characteristic measurement apparatus can measure the temperature at each position of the optical fiber by measuring the scattered light due to Brillouin scattering and measuring the Brillouin Frequency Shift (BFS).

For example, as an optical fiber characteristic measurement apparatus,

Patent Literature (PTL) 1 discloses a BOCDR type optical fiber characteristic measurement apparatus that can reduce the time required to measure the characteristics of an optical fiber.

CITATION LIST

Patent Literature

PTL 1: JP 6773091 B2

SUMMARY

PTL 1 discloses measuring the Brillouin frequency shift and converting the measured Brillouin frequency shift amount into temperature. However, PTL 1 is unable to measure humidity based on the Brillouin frequency shift.

It would therefore be helpful to provide an optical fiber characteristic measurement system and a humidity measuring method that are capable of measuring humidity based on the Brillouin frequency shift.

An optical fiber characteristic measurement system according to several embodiments includes a first optical fiber, a second optical fiber having refractive index characteristics different from those of the first optical fiber, and an optical fiber characteristic measurement apparatus configured to measure a first Brillouin frequency shift, which is a Brillouin frequency shift of Brillouin scattered light from the first optical fiber, and a second Brillouin frequency shift, which is a Brillouin frequency shift of Brillouin scattered light from the second optical fiber, wherein the optical fiber characteristic measurement apparatus is configured to measure humidity at a predetermined position based on the first Brillouin frequency shift and the second Brillouin frequency shift. According to this optical fiber characteristic measurement system, humidity can be measured based on the Brillouin frequency shift.

In the optical fiber characteristic measurement system according to one embodiment, the optical fiber characteristic measurement apparatus may be configured to measure the humidity at the predetermined position by solving simultaneous equations comprising a first relational equation and a second relational equation, the first relational equation being a relational equation relating the first Brillouin frequency shift to temperature and humidity, the second relational equation being a relational equation relating the second Brillouin frequency shift to temperature and humidity. This configuration makes it possible to calculate the humidity easily by solving the simultaneous equations consisting of the first relational equation and the second relational equation.

In the optical fiber characteristic measurement system according to one embodiment, the first optical fiber may have a larger change in refractive index upon moisture absorption than the second optical fiber does. This makes it possible to measure the Brillouin frequency shift using two optical fibers that have different changes in refractive index upon absorbing moisture.

In the optical fiber characteristic measurement system according to one embodiment, the optical fiber characteristic measurement apparatus may be configured to control the predetermined position by adjusting a modulation frequency of light emitted to the first optical fiber and a modulation frequency of light emitted to the second optical fiber. This configuration enables the predetermined position at which humidity is measured to be changed continuously.

The optical fiber characteristic measurement system according to one embodiment may further comprise a switch configured to switch the optical fiber characteristic measurement apparatus between connection to the first optical fiber and to the second optical fiber. As a result, when the first Brillouin frequency shift and the second Brillouin frequency shift are at approximately the same frequency, the first Brillouin frequency shift and the second Brillouin frequency shift can be measured sequentially by switching the connection destination of the optical fiber characteristic measurement apparatus.

In the optical fiber characteristic measurement system according to one embodiment, the optical fiber characteristic measurement apparatus may measure the first Brillouin frequency shift while the optical fiber characteristic measurement apparatus is connected by the switch to the first optical fiber, and measure the second Brillouin frequency shift while the optical fiber characteristic measurement apparatus is connected by the switch to the second optical fiber. This configuration makes it possible to switch the connection of the optical fiber characteristic measurement apparatus and measure the first Brillouin frequency shift and the second Brillouin frequency shift sequentially.

The optical fiber characteristic measurement system according to one embodiment may further comprise an optical coupler configured to split pump light emitted by the optical fiber characteristic measurement apparatus and emit the pump light to the first optical fiber and the second optical fiber. This configuration enables simultaneous measurement of the first Brillouin frequency shift and the second Brillouin frequency shift.

In the optical fiber characteristic measurement system according to one embodiment, the optical fiber characteristic measurement apparatus may be configured to measure the first Brillouin frequency shift and the second Brillouin frequency shift simultaneously. This configuration can reduce the measurement time.

The optical fiber characteristic measurement system according to one embodiment may comprise a first optical fiber characteristic measurement apparatus and a second optical fiber characteristic measurement apparatus as the optical fiber characteristic measurement apparatus, the first optical fiber characteristic measurement apparatus may be connected to the first optical fiber and measure the first Brillouin frequency shift, and the second optical fiber characteristic measurement apparatus may be connected to the second optical fiber and measure the second Brillouin frequency shift. As a result, when the first Brillouin frequency shift and the second Brillouin frequency shift are significantly different from each other, measurements can be made by changing the device configuration of the first optical fiber characteristic measurement apparatus and the device configuration of the second optical fiber characteristic measurement apparatus.

A humidity measurement method according to several embodiments is a humidity measurement method in an optical fiber characteristic measurement system comprising a first optical fiber, a second optical fiber having refractive index characteristics different from those of the first optical fiber, and an optical fiber characteristic measurement apparatus, the humidity measurement method comprising using the optical fiber characteristic measurement apparatus to measure a first Brillouin frequency shift, which is a Brillouin frequency shift of Brillouin scattered light from the first optical fiber, measure a second Brillouin frequency shift, which is a Brillouin frequency shift of Brillouin scattered light from the second optical fiber, and measure humidity at a predetermined position based on the first Brillouin frequency shift and the second Brillouin frequency shift. According to this humidity measurement method, humidity can be measured based on the Brillouin frequency shift.

According to the present disclosure, an optical fiber characteristic measurement system and a humidity measuring method that are capable of measuring humidity based on the Brillouin frequency shift can be provided.

BRIEF DESCRIPTION OF THE DRAWINGS

In the accompanying drawings:

FIG. 1 is a diagram illustrating a schematic configuration of an optical fiber characteristic measurement system according to a first embodiment;
FIG. 2 is a diagram illustrating a schematic configuration of the optical fiber characteristic measurement apparatus illustrated in FIG. 1;
FIG. 3 is a diagram illustrating a state in which a Brillouin frequency shift is measured in an optical fiber characteristic measurement system according to the first embodiment;
FIG. 4 is a diagram illustrating a schematic configuration of an optical fiber characteristic measurement system according to a second embodiment;
FIG. 5 is a diagram illustrating a state in which a Brillouin frequency shift is measured in an optical fiber characteristic measurement system according to the second embodiment;
FIG. 6 is a diagram illustrating a schematic configuration of an optical fiber characteristic measurement system according to a third embodiment; and
FIG. 7 is a diagram illustrating a state in which a Brillouin frequency shift is measured in an optical fiber characteristic measurement system according to the third embodiment.

DETAILED DESCRIPTION

Embodiments of the present disclosure are described below with reference to the drawings.

(First Embodiment)

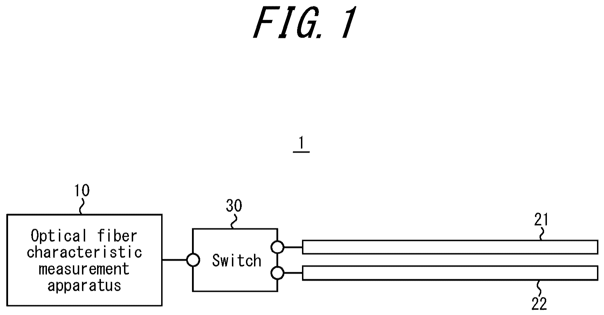

FIG. 1 is a diagram illustrating a schematic configuration of an optical fiber characteristic measurement system 1 according to a first embodiment. The optical fiber characteristic measurement system 1 includes an optical fiber characteristic measurement apparatus 10, a first optical fiber 21, a second optical fiber 22, and a switch 30.

The optical fiber characteristic measurement apparatus 10 can be connected to the first optical fiber 21 or the second optical fiber 22 via the switch 30.

The optical fiber characteristic measurement apparatus 10 is an apparatus that can measure Brillouin scattered light from an optical fiber. The Brillouin scattered light includes a Brillouin gain spectrum. The Brillouin gain spectrum includes a frequency component at approximately 11 GHz. The optical fiber characteristic measurement apparatus 10 can measure the Brillouin frequency shift, which is the amount of shift of the peak of the Brillouin gain spectrum.

The optical fiber characteristic measurement apparatus 10 may be an apparatus that measures the characteristics of the first optical fiber 21 or the second optical fiber 22 to be measured by, for example, Brillouin optical correlation domain reflectometry (BOCDR).

The first optical fiber 21 is an optical fiber that can be used as a sensor for measuring humidity. The first optical fiber 21 can also be used as a sensor for measuring temperature. The first optical fiber 21 may be attached to or embedded in the object for which humidity is to be measured. The object to be measured may be, for example, a building, a road, a bridge, a dam, a tunnel, an aircraft, or the like.

The second optical fiber 22 is an optical fiber that can be used as a sensor for measuring humidity. The second optical fiber 22 can also be used as a sensor for measuring temperature. The second optical fiber 22 may be attached to or embedded in the object to be measured alongside the first optical fiber 21.

The first optical fiber 21 and the second optical fiber 22 have different refractive index characteristics. For example, the first optical fiber 21 may be an optical fiber having a larger change in refractive index upon moisture absorption than the second optical fiber 22 does.

The first optical fiber 21 may be, for example, an optical fiber using PMMA (acrylic resin). Optical fibers using PMMA are hygroscopic and therefore undergo a large change in refractive index upon absorbing moisture.

The second optical fiber 22 may be, for example, a glass optical fiber commonly used for communications. Glass optical fibers that are commonly used for communications have almost no hygroscopicity, and therefore the change in refractive index upon absorbing moisture is small.

The switch 30 can switch the optical fiber characteristic measurement apparatus 10 between connection to the first optical fiber 21 and to the second optical fiber 22. The switch 30 may be a switch having any configuration that can switch the optical fiber characteristic measurement apparatus 10 between connection to the first optical fiber 21 and to the second optical fiber 22.

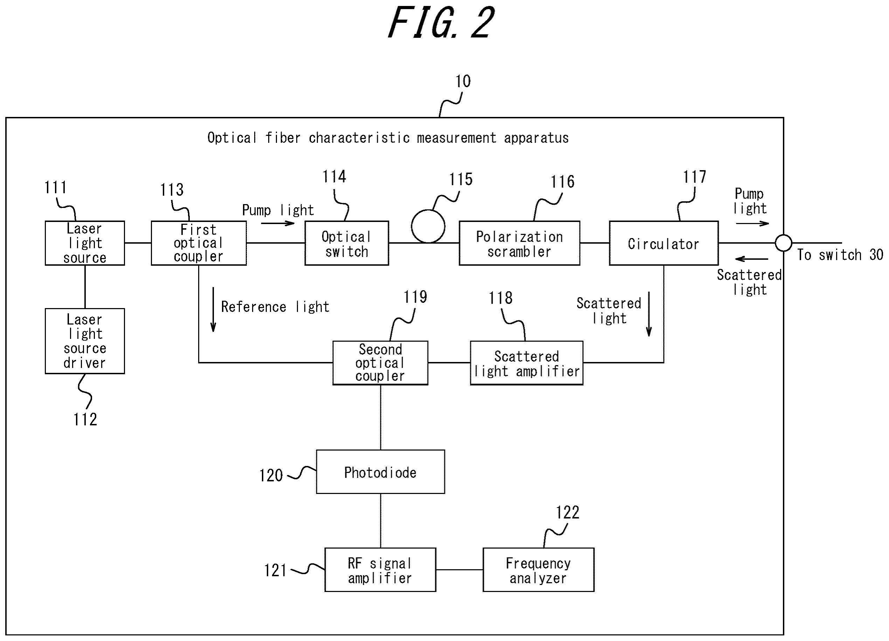

FIG. 2 is a diagram illustrating a schematic configuration of the optical fiber characteristic measurement apparatus 10. In the present embodiment, an example will be described in which the optical fiber characteristic measurement apparatus 10 is a device that measures the characteristics of the first optical fiber 21 or the second optical fiber 22 by BOCDR.

The optical fiber characteristic measurement apparatus 10 includes a laser light source 111, a laser light source driver 112, a first optical coupler 113, an optical switch 114, a delay fiber 115, a polarization scrambler 116, a circulator 117, a scattered light amplifier 118, a second optical coupler 119, a photodiode 120, an RF (Radio Frequency) signal amplifier 121, and a frequency analyzer 122.

The laser light source 111 outputs a laser light. The laser light source 111 may be, for example, a laser diode. The laser light source 111 can output a frequency-modulated laser light. The laser light outputted from the laser light source 111 is frequency modulated by the laser light source driver 112.

The laser light source 111 outputs the frequency-modulated laser light to the first optical coupler 113.

The laser light source driver 112 supplies a sine wave signal to the laser light source 111 to frequency-modulate the laser light outputted by the laser light source 111. The laser light source driver 112 may be a circuit with any configuration that can frequency-modulate the laser light outputted by the laser light source 111.

The first optical coupler 113 splits the laser light supplied from the laser light source 111 into pump light and reference light. The first optical coupler 113 outputs the pump light to the optical switch 114. The first optical coupler 113 outputs the reference light to the second optical coupler 119.

The optical switch 114 chops the pump light supplied from the first optical coupler 113 to generate pulsed light. The optical switch 114 outputs the pump light, which has become pulsed light, to the delay fiber 115.

The delay fiber 115 delays the pump light supplied from the optical switch 114 and outputs the result to the polarization scrambler 116.

The polarization scrambler 116 changes the polarization state of the pump light supplied from the delay fiber 115 to unpolarized light and outputs the result to the circulator 117.

The circulator 117 emits the pump light supplied from the polarization scrambler 116 to the first optical fiber 21 or the second optical fiber 22 via the switch 30. Upon the pump light being emitted to the first optical fiber 21, the first optical fiber 21 generates Brillouin scattered light. The circulator 117 outputs the Brillouin scattered light incident from the first optical fiber 21 to the scattered light amplifier 118. Upon the pump light being emitted to the second optical fiber 22, the second optical fiber 22 generates Brillouin scattered light. The circulator 117 outputs the Brillouin scattered light incident from the second optical fiber 22 to the scattered light amplifier 118.

The scattered light amplifier 118 amplifies the Brillouin scattered light supplied from the circulator 117 and outputs the result to the second optical coupler 119. The scattered light amplifier 118 may be, for example, an Erbium Doped Fiber Amplifier (EDFA).

The second optical coupler 119 combines the Brillouin scattered light supplied from the scattered light amplifier 118 and the reference light supplied from the first optical coupler 113 and outputs the result to the photodiode 120.

The photodiode 120 receives the combined light supplied from the second optical coupler 119 and performs heterodyne detection. The photodiode 120 converts the received light into an electrical signal and outputs the result to the RF signal amplifier 121.

The RF signal amplifier 121 amplifies the electrical signal supplied from the photodiode 120 and outputs the result to the frequency analyzer 122.

The frequency analyzer 122 performs frequency analysis on the electrical signal supplied from the RF signal amplifier 121, that is, the electrical signal corresponding to the light combined by the second optical coupler 119.

The electrical signal supplied to the frequency analyzer 122 from the RF signal amplifier 121 includes a Brillouin gain spectrum due to the Brillouin scattered light. The Brillouin gain spectrum includes a frequency component at approximately 11 GHz.

The frequency analyzer 122 measures the Brillouin frequency shift, which is the amount of shift of the peak of the Brillouin gain spectrum. The Brillouin frequency shift depends to the first order on the temperature and humidity of the first optical fiber 21 or the second optical fiber 22.

The optical fiber characteristic measurement apparatus 10 measures the Brillouin frequency shift of the Brillouin scattered light from the first optical fiber 21 while being connected to the first optical fiber 21 by the switch 30. Hereinafter, the Brillouin frequency shift of the Brillouin scattered light from the first optical fiber 21 may be referred to as the "first Brillouin frequency shift".

The optical fiber characteristic measurement apparatus 10 also measures the Brillouin frequency shift of the Brillouin scattered light from the second optical fiber 22 while being connected to the second optical fiber 22 by the switch 30. Hereinafter, the Brillouin frequency shift of the Brillouin scattered light from the second optical fiber 22 may be referred to as the "second Brillouin frequency shift".

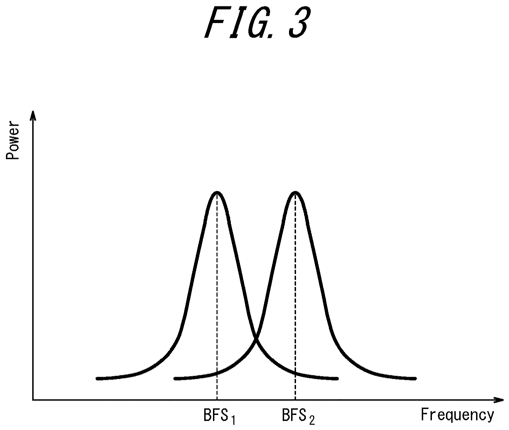

FIG. 3 illustrates an example of how the first Brillouin frequency shift $BFS_1$ and the second Brillouin frequency shift $BFS_2$ are measured in the optical fiber characteristic measurement system 1 according to the first embodiment. In the example illustrated in FIG. 3, the first Brillouin frequency shift $BFS_1$ measured using the first optical fiber 21 and the second Brillouin frequency shift $BFS_2$ measured using the second optical fiber 22 are of approximately the same frequency, but by switching the switch 30 to perform the measurement, the optical fiber characteristic measurement apparatus 10 can measure the first Brillouin frequency shift $BFS_1$ and the second Brillouin frequency shift $BFS_2$ sequentially.

Upon measuring the first Brillouin frequency shift and the second Brillouin frequency shift, the optical fiber characteristic measurement apparatus 10 can measure the humidity at a predetermined position based on the measured first Brillouin frequency shift and second Brillouin frequency shift.

First, how to control the predetermined position at which humidity is measured will be described.

The optical fiber characteristic measurement apparatus 10 can control the predetermined position by adjusting the modulation frequency of light emitted to the first optical fiber 21 and the modulation frequency of light emitted to the second optical fiber 22. The optical fiber characteristic measurement apparatus 10 adjusts the modulation frequency of the light emitted to the first optical fiber 21 and the second optical fiber 22 by changing the frequency of the sine wave signal that the laser light source driver 112 supplies to the laser light source 111.

The predetermined position z at which the humidity is measured is expressed, for example, by the following Equations (1) and (2). The predetermined position z is a position that, with respect to the end of the first optical fiber 21 on the switch 30 side, is a distance z towards the other end of the first optical fiber 21. Alternatively, the predetermined position z is a position that, with respect to the end of the second optical fiber 22 on the switch 30 side, is a distance z towards the other end of the second optical fiber 22.

$$ z = -\frac{L_d}{2} + m d_m \quad (1) $$

$$ d_m = \frac{v_g}{2 f_m} \quad (2) $$

In the Equations, $L_d$ is the length of the delay fiber 115, m is an integer, $v_g$ is the speed of light in the first optical fiber 21 or the speed of light in the second optical fiber 22, and $f_m$ is the modulation frequency.

The optical fiber characteristic measurement apparatus 10 can continuously change the predetermined position z, which is the target for measuring humidity, by adjusting the modulation frequency.

In the above Equation (2), the value of $v_g$ is different between the first optical fiber 21 and the second optical fiber 22. Therefore, the optical fiber characteristic measurement apparatus 10 adjusts the modulation frequency between the cases of measuring the first Brillouin frequency shift using the first optical fiber 21 and measuring the second Brillouin frequency shift using the second optical fiber 22, so that the predetermined position is approximately the same position.

Next, the way in which the optical fiber characteristic measurement apparatus 10 measures humidity based on the measured first Brillouin frequency shift and second Brillouin frequency shift will be described.

Since the first Brillouin frequency shift depends to the first order on the temperature and humidity of the first optical fiber 21, it can be expressed as a first relational equation, which is a relational equation relating the first Brillouin frequency shift to the temperature and humidity, for example, as in the following Equation (3).

$$BFS_1 = a_1 \times T + b_1 \times H \qquad (3)$$

In Equation (3), $BFS_1$ is the first Brillouin frequency shift, $a_1$ is a temperature-dependent parameter of the first optical fiber 21, T is the temperature, $b_1$ is a humidity-dependent parameter of the first optical fiber 21, and H is the humidity.

Since the second Brillouin frequency shift depends to the first order on the temperature and humidity of the second optical fiber 22, it can be expressed as a second relational equation, which is a relational equation relating the second Brillouin frequency shift to the temperature and humidity, for example, as in the following Equation (4).

$$BFS_2 = a_2 \times T + b_2 \times H \qquad (4)$$

In Equation (4), $BFS_2$ is the second Brillouin frequency shift, $a_2$ is a temperature-dependent parameter of the second optical fiber 22, T is the temperature, $b_2$ is a humidity-dependent parameter of the second optical fiber 22, and H is the humidity.

In the above Equation (3), $a_1$ and $b_1$ are parameters that depend on the characteristics of the first optical fiber 21, and in the above Equation (4), $a_2$ and $b_2$ are parameters that depend on the characteristics of the second optical fiber 22.

For example, if the first optical fiber 21 experiences a large change in refractive index upon absorbing moisture, $b_1$ in the above Equation (3) will be a large value. Furthermore, for example, if the refractive index of the second optical fiber 22 hardly changes even upon absorbing moisture, $b_2$ in the above formula (4) will be a value close to zero.

Since the first optical fiber 21 and the second optical fiber 22 are arranged side by side at approximately the same location, in the above Equations (3) and (4), the temperature T is the same temperature and the humidity H is the same humidity.

Therefore, the optical fiber characteristic measurement apparatus 10 can calculate the temperature and humidity at a specified location by solving the simultaneous equations consisting of the first relational equation illustrated in the above-described Equation (3) and the second relational equation illustrated in the above-described Equation (4). That is, the optical fiber characteristic measurement apparatus 10 can measure the humidity at a predetermined position by solving the simultaneous equations consisting of the first relational equation and the second relational equation.

(Second Embodiment)

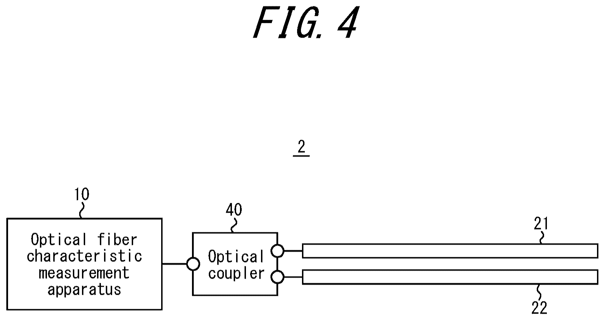

FIG. 4 is a diagram illustrating a schematic configuration of an optical fiber characteristic measurement system 2 according to a second embodiment. The optical fiber characteristic measurement system 2 includes an optical fiber characteristic measurement apparatus 10, a first optical fiber 21, a second optical fiber 22, and an optical coupler 40.

The differences between the optical fiber characteristic measurement system 2 of the second embodiment and the optical fiber characteristic measurement system 1 of the first embodiment will be mainly described, and descriptions of points in common or similar to the optical fiber characteristic measurement system 1 according to the first embodiment will be omitted as appropriate.

The optical fiber characteristic measurement system 2 according to the second embodiment differs from the optical fiber characteristic measurement system 1 according to the first embodiment mainly by inclusion of the optical coupler 40 instead of the switch 30.

The optical coupler 40 splits the pump light emitted by the optical fiber characteristic measurement apparatus 10 and emits the pump light to the first optical fiber 21 and the second optical fiber 22.

In the optical fiber characteristic measurement system 2 according to the second embodiment, the optical fiber characteristic measurement apparatus 10 simultaneously emits the pump light to the first optical fiber 21 and the second optical fiber 22 via the optical coupler 40. The optical fiber characteristic measurement apparatus 10 can then simultaneously measure the first Brillouin frequency shift of the first optical fiber 21 and the second Brillouin frequency shift of the second optical fiber 22.

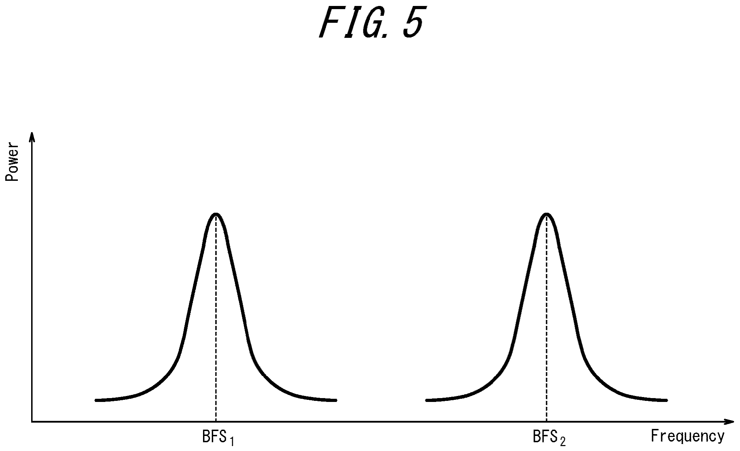

FIG. 5 illustrates an example of how the first Brillouin frequency shift $BFS_1$ and the second Brillouin frequency shift $BFS_2$ are measured in the optical fiber characteristic measurement system 2 according to the second embodiment. In the example illustrated in FIG. 5, the first Brillouin frequency shift $BFS_1$ measured using the first optical fiber 21 and the second Brillouin frequency shift $BFS_2$ measured using the second optical fiber 22 have similar wavelength bands but are sufficiently separated in frequency. In such a case, the two waveforms can be separated, and the optical fiber characteristic measurement apparatus 10 can measure the first Brillouin frequency shift $BFS_1$ and the second Brillouin frequency shift $BFS_2$ simultaneously.

In the optical fiber characteristic measurement system 2 according to the second embodiment, the optical fiber characteristic measurement apparatus 10 can measure the humidity at a predetermined position based on the measured first Brillouin frequency shift and second Brillouin frequency shift, similar to the optical fiber characteristic measurement system 1 according to the first embodiment.

(Third Embodiment)

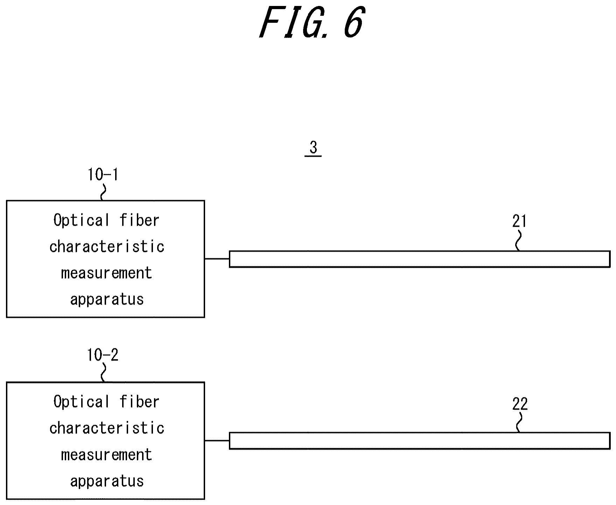

FIG. 6 is a diagram illustrating a schematic configuration of an optical fiber characteristic measurement system 3 according to a third embodiment. The optical fiber characteristic measurement system 3 includes an optical fiber characteristic measurement apparatus 10-1, an optical fiber characteristic measurement apparatus 10-2, a first optical fiber 21, and a second optical fiber 22.

The differences between the optical fiber characteristic measurement system 3 of the third embodiment and the optical fiber characteristic measurement system 1 of the first embodiment will be mainly described, and descriptions of points in common or similar to the optical fiber characteristic measurement system 1 according to the first embodiment will be omitted as appropriate.

The optical fiber characteristic measurement system 3 according to the third embodiment differs mainly from the optical fiber characteristic measurement system 1 of the first embodiment by including the two optical fiber characteristic measurement apparatuses 10-1 and 10-2 and not including the switch 30.

The optical fiber characteristic measurement apparatus 10-1 (corresponding to the "first optical fiber characteristic measurement apparatus" in the claims) is connected to the first optical fiber 21. The optical fiber characteristic measurement apparatus 10-1 measures the first Brillouin frequency shift of the first optical fiber 21.

The optical fiber characteristic measurement apparatus 10-2 (corresponding to the "second optical fiber characteristic measurement apparatus" in the claims) is connected to the second optical fiber 22. The optical fiber characteristic measurement apparatus 10-2 measures the second Brillouin frequency shift of the second optical fiber 22.

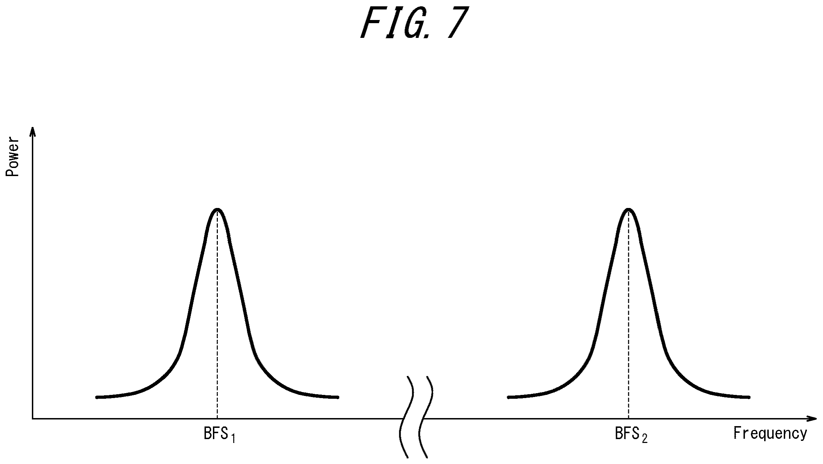

FIG. 7 illustrates an example of how the first Brillouin frequency shift $BFS_1$ and the second Brillouin frequency shift $BFS_2$ are measured in the optical fiber characteristic measurement system 3 according to the third embodiment. In the example illustrated in FIG. 7, the first Brillouin frequency shift $BFS_1$ measured using the first optical fiber 21 and the second Brillouin frequency shift $BFS_2$ measured using the second optical fiber 22 are at significantly separated frequencies and do not overlap in wavelength band. In such a case in which the first Brillouin frequency shift and the second Brillouin frequency shift are significantly separated, it may be necessary to change the optical fiber characteristic measurement apparatus 10-1 and the optical fiber characteristic measurement apparatus 10-2 to different apparatus configurations. By adopting a configuration that uses the two optical fiber characteristic measurement apparatuses 10-1 and 10-2, the apparatus configuration can be easily changed.

In the optical fiber characteristic measurement system 3 according to the third embodiment, the optical fiber characteristic measurement apparatus 10-1 can measure the humidity at a predetermined position based on the first Brillouin frequency shift measured by the optical fiber characteristic measurement apparatus 10-1 and the second Brillouin frequency shift measured by the optical fiber characteristic measurement apparatus 10-2. In this case, the optical fiber characteristic measurement apparatus 10-1 may acquire information on the second Brillouin frequency shift from the optical fiber characteristic measurement apparatus 10-2 to which it is communicably connected.

Alternatively, the optical fiber characteristic measurement apparatus 10-2 may acquire information on the first Brillouin frequency shift from the optical fiber characteristic measurement apparatus 10-1 and measure the humidity at a predetermined position based on the first Brillouin frequency shift measured by the optical fiber characteristic measurement apparatus 10-1 and the second Brillouin frequency shift measured by the optical fiber characteristic measurement apparatus 10-2.

According to the optical fiber characteristic measurement systems 1 to 3 of the first to third embodiments described above, humidity can be measured based on the Brillouin frequency shift. In greater detail, the optical fiber characteristic measurement systems 1 to 3 according to the present disclosure include the first optical fiber 21, the second optical fiber 22 having refractive index characteristics different from those of the first optical fiber 21, and the optical fiber characteristic measurement apparatus 10 configured to measure the first Brillouin frequency shift, which is the Brillouin frequency shift of Brillouin scattered light from the first optical fiber 21, and the second Brillouin frequency shift, which is the Brillouin frequency shift of Brillouin scattered light from the second optical fiber 22. The optical fiber characteristic measurement apparatus 10 then measures the humidity at a predetermined position based on the first Brillouin frequency shift and the second Brillouin frequency shift. In this way, by measuring the first Brillouin frequency shift and the second Brillouin frequency shift using the first optical fiber 21 and the second optical fiber 22, which have different refractive index characteristics, the optical fiber characteristic measurement systems 1 to 3 according to the first to third embodiments can measure humidity based on the first Brillouin frequency shift and the second Brillouin frequency shift.

It will be apparent to those skilled in the art that the present disclosure may be realized in certain forms other than the above-described embodiments without departing from the spirit or essential characteristics of the present disclosure. Accordingly, the foregoing description is merely illustrative and is not limiting. The scope of the disclosure is defined by the appended claims, not by the foregoing description. Among all modifications, those within a range of equivalents to the present disclosure shall be considered as being included in the present disclosure.

For example, the arrangement and number of each of the above-mentioned components are not limited to those illustrated in the above description and the drawings. The arrangement and number of each component may be configured freely as long as the corresponding function can be realized.

For example, the optical fiber characteristic measurement apparatus 10 has been described as measuring the first Brillouin frequency shift of the first optical fiber 21 and the second Brillouin frequency shift of the second optical fiber 22 by BOCDR, but this configuration is not limiting. The optical fiber characteristic measurement apparatus 10 may measure the first Brillouin frequency shift of the first optical fiber 21 and the second Brillouin frequency shift of the second optical fiber 22, by, for example, Brillouin optical correlation domain analysis (BOCDA).

Examples of some embodiments of the present disclosure are described below. However, it should be noted that the embodiments of the present disclosure are not limited to these examples.

[Appendix 1] An optical fiber characteristic measurement system comprising:

a first optical fiber;
a second optical fiber having refractive index characteristics different from those of the first optical fiber; and
an optical fiber characteristic measurement apparatus configured to measure a first Brillouin frequency shift, which is a Brillouin frequency shift of Brillouin scattered light from the first optical fiber, and a second Brillouin frequency shift, which is a Brillouin frequency shift of Brillouin scattered light from the second optical fiber, wherein
the optical fiber characteristic measurement apparatus is configured to measure humidity at a predetermined position based on the first Brillouin frequency shift and the second Brillouin frequency shift.

[Appendix 2] The optical fiber characteristic measurement system according to appendix 1, wherein the optical fiber characteristic measurement apparatus is configured to measure the humidity at the predetermined position by solving simultaneous equations comprising a first relational equation and a second relational equation, the first relational equation being a relational equation relating the first Brillouin frequency shift to temperature and humidity, the second relational equation being a relational equation relating the second Brillouin frequency shift to temperature and humidity.

[Appendix 3] The optical fiber characteristic measurement system according to appendix 1 or 2, wherein the first optical fiber has a larger change in refractive index upon moisture absorption than the second optical fiber does.

[Appendix 4] The optical fiber characteristic measurement system according to any one of appendices 1 to 3, wherein the optical fiber characteristic measurement apparatus is configured to control the predetermined position by adjusting a modulation frequency of light emitted to the first optical fiber and a modulation frequency of light emitted to the second optical fiber.

[Appendix 5] The optical fiber characteristic measurement system according to any one of appendices 1 to 4, further comprising a switch configured to switch the optical fiber characteristic measurement apparatus between connection to the first optical fiber and to the second optical fiber.

[Appendix 6] The optical fiber characteristic measurement system according to any one of appendices 1 to 5, wherein
the optical fiber characteristic measurement apparatus

measures the first Brillouin frequency shift while the optical fiber characteristic measurement apparatus is connected by the switch to the first optical fiber, and
measures the second Brillouin frequency shift while the optical fiber characteristic measurement apparatus is connected by the switch to the second optical fiber.

[Appendix 7] The optical fiber characteristic measurement system according to any one of appendices 1 to 6, further comprising an optical coupler configured to split pump light emitted by the optical fiber characteristic measurement apparatus and emit the pump light to the first optical fiber and the second optical fiber.

[Appendix 8] The optical fiber characteristic measurement system according to any one of appendices 1 to 7, wherein the optical fiber characteristic measurement apparatus is configured to measure the first Brillouin frequency shift and the second Brillouin frequency shift simultaneously.

[Appendix 9] The optical fiber characteristic measurement system according to any one of appendices 1 to 8, comprising

a first optical fiber characteristic measurement apparatus and a second optical fiber characteristic measurement apparatus as the optical fiber characteristic measurement apparatus, wherein
the first optical fiber characteristic measurement apparatus is connected to the first optical fiber and measures the first Brillouin frequency shift, and
the second optical fiber characteristic measurement apparatus is connected to the second optical fiber and measures the second Brillouin frequency shift.

[Appendix 10] A humidity measurement method in an optical fiber characteristic measurement system comprising a first optical fiber, a second optical fiber having refractive index characteristics different from those of the first optical fiber, and an optical fiber characteristic measurement apparatus, the humidity measurement method comprising:

using the optical fiber characteristic measurement apparatus to measure a first Brillouin frequency shift, which is a Brillouin frequency shift of Brillouin scattered light from the first optical fiber;

measure a second Brillouin frequency shift, which is a Brillouin frequency shift of Brillouin scattered light from the second optical fiber; and

measure humidity at a predetermined position based on the first Brillouin frequency shift and the second Brillouin frequency shift.

**Claims**

1. An optical fiber characteristic measurement system comprising:

   a first optical fiber;

   a second optical fiber having refractive index characteristics different from those of the first optical fiber; and

   an optical fiber characteristic measurement apparatus configured to measure a first Brillouin frequency shift, which is a Brillouin frequency shift of Brillouin scattered light from the first optical fiber, and a second Brillouin frequency shift, which is a Brillouin frequency shift of Brillouin scattered light from the second optical fiber, wherein

   the optical fiber characteristic measurement apparatus is configured to measure humidity at a predetermined position based on the first Brillouin frequency shift and the second Brillouin frequency shift.

2. The optical fiber characteristic measurement system according to claim 1, wherein the optical fiber characteristic measurement apparatus is configured to measure the humidity at the predetermined position by solving simultaneous equations comprising a first relational equation and a second relational equation, the first relational equation being a relational equation relating the first Brillouin frequency shift to temperature and humidity, the second relational equation being a relational equation relating the second Brillouin frequency shift to temperature and humidity.

3. The optical fiber characteristic measurement system according to claim 1, wherein the first optical fiber has a larger change in refractive index upon moisture absorption than the second optical fiber does.

4. The optical fiber characteristic measurement system according to claim 1, wherein the optical fiber characteristic measurement apparatus is configured to control the predetermined position by adjusting a modulation frequency of light emitted to the first optical fiber and a modulation frequency of light emitted to the second optical fiber.

5. The optical fiber characteristic measurement system according to claim 1, further comprising a switch configured to switch the optical fiber characteristic measurement apparatus between connection to the first optical fiber and to the second optical fiber.

6. The optical fiber characteristic measurement system according to claim 5, wherein
   the optical fiber characteristic measurement apparatus

   measures the first Brillouin frequency shift while the optical fiber characteristic measurement apparatus is connected by the switch to the first optical fiber, and

   measures the second Brillouin frequency shift while the optical fiber characteristic measurement apparatus is connected by the switch to the second optical fiber.

7. The optical fiber characteristic measurement system according to claim 1, further comprising an optical coupler configured to split pump light emitted by the optical fiber characteristic measurement apparatus and emit the pump light to the first optical fiber and the second optical fiber.

8. The optical fiber characteristic measurement system according to claim 7, wherein the optical fiber characteristic measurement apparatus is configured to measure the first Brillouin frequency shift and the second Brillouin frequency shift simultaneously.

9. The optical fiber characteristic measurement system according to claim 1, comprising

   a first optical fiber characteristic measurement apparatus and a second optical fiber characteristic measurement apparatus as the optical fiber characteristic measurement apparatus, wherein

   the first optical fiber characteristic measurement apparatus is connected to the first optical fiber and measures the first Brillouin frequency shift, and

   the second optical fiber characteristic measurement apparatus is connected to the second optical fiber and measures the second Brillouin frequency shift.

**10.** A humidity measurement method in an optical fiber characteristic measurement system comprising a first optical fiber, a second optical fiber having refractive index characteristics different from those of the first optical fiber, and an optical fiber characteristic measurement apparatus, the humidity measurement method comprising:

using the optical fiber characteristic measurement apparatus to

measure a first Brillouin frequency shift, which is a Brillouin frequency shift of Brillouin scattered light from the first optical fiber;
measure a second Brillouin frequency shift, which is a Brillouin frequency shift of Brillouin scattered light from the second optical fiber; and
measure humidity at a predetermined position based on the first Brillouin frequency shift and the second Brillouin frequency shift.

# FIG. 1

# FIG. 2

Optical fiber characteristic measurement apparatus (10)

- 111 Laser light source
- 112 Laser light source driver
- 113 First optical coupler
- 114 Optical switch — Pump light
- 115
- 116 Polarization scrambler
- 117 Circulator — Pump light / Scattered light — To switch 30
- 118 Scattered light amplifier — Scattered light
- 119 Second optical coupler — Reference light
- 120 Photodiode
- 121 RF signal amplifier
- 122 Frequency analyzer

EP 4 726 334 A1

FIG. 3

# FIG. 4

2

10

Optical fiber
characteristic
measurement
apparatus

40

Optical
coupler

21

22

EP 4 726 334 A1

# FIG. 5

# *FIG. 6*

*FIG. 7*

Europäisches
Patentamt

European
Patent Office

Office européen
des brevets

# EUROPEAN SEARCH REPORT

**Application Number**

EP 25 20 3895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/063209 A1 (NAKAMICHI MASANORI [JP] ET AL) 4 March 2021 (2021-03-04)<br>* paragraph [0015] - paragraph [0017] *<br>* paragraph [0035] - paragraph [0038] *<br>* paragraph [0066] - paragraph [0088] *<br>* figures 1, 7-11 * | 1-10 | INV.<br>G01D5/353 |
| X | CN 110 887 527 A (UNIV XIAMEN) 17 March 2020 (2020-03-17)<br>* paragraph [0006] - paragraph [0016] *<br>* paragraph [0023] - paragraph [0034] *<br>* figures 1-2 * | 1,4,10 | |
| X | SCHREIER ANDY ET AL: "Comparison of solution approaches for distributed humidity sensing in perfluorinated graded-index polymer optical fibers", SPIE PROCEEDINGS; [PROCEEDINGS OF SPIE ISSN 0277-786X], SPIE, US, vol. 11028, 11 April 2019 (2019-04-11), pages 1102808-1102808, XP060123354, DOI: 10.1117/12.2522307 ISBN: 978-1-5106-3673-6<br>* abstract; figure 4 *<br>* 1. Introduction *<br>* 2. Sensing methods 2.3 Brillouin frequency shift * | 1,4,10 | TECHNICAL FIELDS SEARCHED (IPC)<br><br>G01D<br>G01N |

The present search report has been drawn up for all claims

| Place of search | Date of completion of the search | Examiner |
|---|---|---|
| Munich | 3 March 2026 | Paraf, Edouard |

EPO FORM 1503 03.82 (P04C01)

## ANNEX TO THE EUROPEAN SEARCH REPORT
## ON EUROPEAN PATENT APPLICATION NO.

EP 25 20 3895

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2026

| Patent document cited in search report | | Publication date | Patent family member(s) | | Publication date |
|---|---|---|---|---|---|
| US 2021063209 | A1 | 04-03-2021 | JP | 7177991 B2 | 25-11-2022 |
| | | | JP | 2021038963 A | 11-03-2021 |
| | | | US | 2021063209 A1 | 04-03-2021 |
| CN 110887527 | A | 17-03-2020 | NONE | | |

EPO FORM P0459

For more details about this annex : see Official Journal of the European Patent Office, No. 12/82

**EP 4 726 334 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024176767 A

- JP 6773091 B